# EUROPÄISCHE PATENTSCHRIFT

(11) **EP 4 327 153 B1**
(45) Veröffentlichungstag und Bekanntmachung des Hinweises auf die Patenterteilung: **16.10.2024**
(21) Anmeldenummer: 22724064.5
(22) Anmeldetag: 21.04.2022
(51) Int. Cl.: G02B 21/00, A61B 90/20, A61B 3/13, G02B 7/28, G06T 7/593, G02B 7/00

(54) **VERFAHREN ZUM BETREIBEN EINES OPERATIONSMIKROSKOPS UND OPERATIONSMIKROSKOP**
METHOD FOR OPERATING A SURGICAL MICROSCOPE, AND SURGICAL MICROSCOPE
PROCÉDÉ DE FONCTIONNEMENT D'UN MICROSOSCOPE CHIRURGICAL ET MICROSOSCOPE CHIRURGICAL

(30) Priorität: 22.04.2021 DE 102021204031
(43) Veröffentlichungstag der Anmeldung: 28.02.2024
(73) Patentinhaber: Carl Zeiss Meditec AG, 07745 Jena (DE)
(72) Erfinder: ROODAKI, Hessam, 81479 München (DE); ESLAMI, Abouzar, 81479 München (DE); SARHAN, Mhd Hasan, 80798 München (DE)
(74) Vertreter: Patentanwälte Bressel und Partner mbB
(86) Internationale Anmeldenummer: PCT/EP2022/060491
(87) Internationale Veröffentlichungsnummer: WO 2022/223663

(56) Entgegenhaltungen:
- CN-A- 111 491 151
- DE-A1- 102014 102 080
- DE-A1- 102018 217 903
- DE-A1- 102018 219 867
- DE-A1- 102019 133 174
- US-A1- 2019 324 252

## Beschreibung

Die Erfindung betrifft ein Verfahren zum Betreiben eines Operationsmikroskops und ein Operationsmikroskop.

Bei der Anwendung eines Operationsmikroskops zur Unterstützung einer chirurgischen Operation müssen vor Beginn des Eingriffs Mikroskopparameter in Abhängigkeit des chirurgischen Zielobjektes (z.B. Limbus des Auges, Gliome bei der Kraniotomie etc.) eingestellt werden. Die Mikroskopparameter betreffen hierbei insbesondere eine Position eines Mikroskopkopfes relativ zum chirurgischen Zielobjekt sowie Parameter einer Abbildungsoptik. Insbesondere betreffen die Mikroskopparameter hierbei einen Fokus bzw. eine Fokusebene, eine Vergrößerung und/oder eine Zentrierung auf das Zielobjekt. Dieser Prozess des Konfigurierens ist in der Regel anspruchsvoll und zeitaufwändig.

Aus der US 2019/0313902 A1 sind ein Verfahren und ein System zum automatischen Zentrieren eines Sichtfelds des Auges eines Patienten unter starker Vergrößerung während einer Augenchirurgie in einer XY-Ebene bekannt. Das Verfahren umfasst das automatische Verschieben der Mitte des Sichtfelds in die Mitte eines kreisförmigen Bildes, das in einem Echtzeitvideosignal erfasst wird, das aus dem Sichtfeld des Auges des Patienten unter starker Vergrößerung während einer Augenoperation erfasst wird.

Aus der US 9 479 759 B2 ist eine optische Stereovorrichtung mit einem Autofokusmerkmal sowie ein entsprechendes Autofokusverfahren für optische Stereovorrichtungen bekannt. Die optische Stereovorrichtung weist Abbildungsmittel auf, die konfiguriert sind, um ein Stereobild eines interessierenden Objekts bereitzustellen, indem ein Bild des rechten Auges und ein Bild des linken Auges und eine Steuereinheit kombiniert werden, die betriebsmäßig mit den Abbildungsmitteln verbunden ist und konfiguriert ist, um das Bild des rechten Auges und das Bild des linken Auges zu empfangen und die Fokusposition der Bildgebungsmittel einzustellen.

Aus der WO 2009/080790 A1 ist ein Verfahren zur Ermittlung des Radius und/oder der Position charakteristischer Augenbestandteile während einer Augenuntersuchung oder Augenbehandlung bekannt. Dabei wird ein digitales Bild zumindest eines Ausschnittes eines Auges mit einer Kamera aufgenommen. Das Bild wird mit ringförmigen Vergleichsobjekten unterschiedlicher Größe so korreliert, dass sich die größte Übereinstimmung zwischen Bild und Vergleichsobjekt beim Zusammentreffen eines ringförmigen Vergleichsobjekts mit einem ringförmigen Dichtesprung gleichen Radius im Bild ergibt. Dann werden die Vergleichsobjekte mit lokal großer Übereinstimmung mit dem digitalen Bild ermittelt und aus diesen Vergleichsobjekten großer Übereinstimmung der Radius und/oder die Position des charakteristischen Augenbestandteils abgeleitet.

Aus der DE 10 2014 102 080 A1 ist ein Bildaufnahmesystem bekannt. Das Bildaufnahmesystem umfasst eine Steuereinrichtung zum automatischen Einstellen mehrerer Bildaufnahmeparameter zur Bildaufnahme. Die Steuereinrichtung ist eingerichtet, um die mehreren Bildaufnahmeparameter durch Anwendung eines Regelsatzes einzustellen. Die Steuereinrichtung ist eingerichtet, um den Regelsatz automatisch zu erlernen oder zu adaptieren.

Aus der DE 10 2018 217 903 A1 sind ein Verfahren und eine Vorrichtung zur Optimierung von Arbeitsabläufen mindestens eines Mikroskops oder Mikroskopsystems bekannt. Das Verfahren und die Vorrichtung umfassen die folgenden Schritte: Ausführen eines Arbeitsablaufes durch ein oder mehrere Komponenten mindestens eines Mikroskops und/oder Mikroskopsystems, wobei der Arbeitsablauf ein Erfassen von ersten Daten umfasst, Anwenden eines trainierten Modells auf die erfassten ersten Daten, und Treffen mindestens einer Entscheidung den Arbeitsablauf betreffend basierend auf dem Anwenden des trainierten Modells.

Aus der DE 10 2018 219 867 A1 sind ein Verfahren und eine Vorrichtung zur Bestimmung einer Fokusposition mittels trainierter Modelle bekannt. Das Verfahren und die Vorrichtung sehen ein Aufnehmen mindestens eines ersten Bildes vor, wobei Bilddaten des mindestens einen aufgenommenen ersten Bildes von mindestens einer ersten Fokusposition bei der Aufnahme des mindestens einen ersten Bildes abhängen, und ein Bestimmen einer zweiten Fokusposition basierend auf einer Analyse des mindestens einen aufgenommenen ersten Bildes mittels eines trainierten Modells.

Aus der CN 111491151 A ist ein mikrochirurgisches dreidimensionales Videowiedergabeverfahren bekannt. Das Verfahren umfasst die Schritte: Erhalten eines Echtzeit-Blickpunktbildes eines mikroskopischen optischen Systems; Erhalten einer Tiefenkarte; Segmentieren der Tiefenkarte und Konstruieren eines ersten Maskenbildes; mehrmaliges Ausführen einer Maskenbildkonstruktion auf dem ersten Maskenbild und Ausführen einer Erweiterungs- und Vereinigungsoperation, um ein viertes Maskenbild zu erhalten; Lösen eines Bildes mit doppeltem Betrachtungspunkt gemäß Maskeninformationen, die in dem vierten Maskenbild enthalten sind, und Rendern und Synthetisieren des Bildes mit doppeltem Betrachtungspunkt, um ein dreidimensionales Bild zu erhalten, das auf einem mikroskopischen Bildsystem angezeigt werden kann; und Ausgeben eines stereoskopischen Videos gemäß dem stereoskopischen Bild, wobei ein auf optischem Fluss basierender Zielerfassungsalgorithmus zum Verfolgen des hinteren Endes eines chirurgischen Instruments in einem Mikrochirurgiefeld verwendet wird und der Arbeitsabstand des optischen Mikroskopiesystems angepasst und aktualisiert wird.

Aus der US 2019 / 0 324 252 A1 ist ein chirurgisches Mikroskopiesystem mit einer automatischen Zoomsteuerung bekannt. Aus der nachveröffentlichten DE 10 2019 133 174 A1 ist ein Verfahren zum kontextsensitiven Weißabgleich bekannt.

Der Erfindung liegt die Aufgabe zu Grunde, ein Verfahren zum Betreiben eines Operationsmikroskops und ein Operationsmikroskop zu schaffen, bei denen Mikroskopparameter automatisiert bestimmt und eingestellt werden können.

Die Aufgabe wird erfindungsgemäß durch ein Verfahren mit den Merkmalen des Patentanspruchs 1 und ein Operationsmikroskop mit den Merkmalen des Patentanspruchs 8 gelöst. Vorteilhafte Ausgestaltungen der Erfindung ergeben sich aus den Unteransprüchen.

Es ist einer der Grundgedanken der Erfindung, mindestens ein trainiertes Maschinenlernverfahren und/oder Computer-Vision-Auswertungsverfahren zu verwenden, um ausgehend von einer erfassten linksseitigen Abbildung und einer erfassten rechtsseitigen Abbildung optimale Mikroskopparameter und/oder eine Änderung der Mikroskopparameter und/oder Steuerbefehle einer Aktorik des Operationsmikroskops zu schätzen. Das trainierte mindestens eine Maschinenlernverfahren identifiziert insbesondere ein (chirurgisches) Zielobjekt in den Abbildungen und schätzt die optimalen Mikroskopparameter und/oder die Änderung der Mikroskopparameter und/oder die Steuerbefehle für dieses Zielobjekt. Das Schätzen erfolgt hierbei insbesondere mit nur einer linksseitigen Abbildung und nur einer rechtsseitigen Abbildung. Beim Identifizieren des Zielobjektes werden eine Art des Zielobjektes und eine Position des Zielobjektes in den Abbildungen identifiziert bzw. bestimmt. Das Zielobjekt kann beispielsweise ein Limbus des Auges, Gliome bei der Kraniotomie etc. sein. Alternativ oder zusätzlich wird das (chirurgische) Zielobjekt insbesondere mittels des Computer-Vision-Auswertungsverfahren in den Abbildungen identifiziert. Das Zielobjekt wird insbesondere durch das Maschinenlernverfahren und/oder das Computer-Vision-Auswertungsverfahren identifiziert, eine vorangehende Anwahl und/oder Auswahl und/oder Markierung des Zielobjektes erfolgt nicht. Die optimalen Mikroskopparameter sind insbesondere in Bezug auf eine Darstellung des identifizierten Zielobjektes in nachfolgend, das heißt, nach Ändern einer Konfiguration des Operationsmikroskops, erfassten Abbildungen optimiert. Anders ausgedrückt: die Mikroskopparameter werden für ein optimales Arbeiten an dem chirurgischen Zielobjekt geschätzt. Die Steuereinrichtung steuert eine Aktorik des Operationsmikroskops zum Einstellen der optimalen Mikroskopparameter und/oder der Änderung der Mikroskopparameter an und erzeugt hierzu Steuerbefehle. Werden von dem mindestens einen trainierten Maschinenlernverfahren bereits Steuerbefehle geschätzt, so werden diese mittels der Steuereinrichtung durch entsprechendes Ansteuern der Aktorik des Operationsmikroskops umgesetzt.

Insbesondere wird ein Verfahren zum Betreiben eines Operationsmikroskops zur Verfügung gestellt, wobei eine linksseitige Abbildung eines Erfassungsbereichs des Operationsmikroskops mittels einer linksseitigen Kamera des Operationsmikroskops erfasst wird, wobei eine rechtsseitige Abbildung des Erfassungsbereichs mittels einer rechtsseitigen Kamera des Operationsmikroskops erfasst wird, wobei die erfasste linkseitige Abbildung und die erfasste rechtsseitige Abbildung mindestens einem mittels einer Steuereinrichtung des Operationsmikroskops bereitgestellten trainierten Maschinenlernverfahren und/oder Computer-Vision-Auswertungsverfahren als Eingangsdaten zugeführt werden, und wobei mittels des mindestens einen trainierten Maschinenlernverfahrens und/oder Computer-Vision-Auswertungsverfahrens ausgehend von den erfassten Abbildungen ein Zielobjekt in den erfassten Abbildungen identifiziert wird und hierfür optimale Mikroskopparameter und/oder eine Änderung von Mikroskopparametern und/oder Steuerbefehle für eine Aktorik des Operationsmikroskops geschätzt werden, wobei mittels der Steuereinrichtung aus den geschätzten optimalen Mikroskopparametern und/oder der geschätzten Änderung der Mikroskopparameter Steuerbefehle für die Aktorik des Operationsmikroskops erzeugt werden und/oder wobei die Aktorik entsprechend den erzeugten und/oder geschätzten Steuerbefehlen angesteuert wird.

Ferner wird insbesondere ein Operationsmikroskop geschaffen, umfassend eine linkseitige Kamera, eingerichtet zum Erfassen einer linkseitigen Abbildung eines Erfassungsbereichs des Operationsmikroskops, eine rechtsseitige Kamera, eingerichtet zum Erfassen einer rechtsseitigen Abbildung des Erfassungsbereichs, eine Aktorik, eingerichtet zum Einstellen einer Konfiguration des Operationsmikroskops gemäß Mikroskopparametern, und eine Steuereinrichtung, wobei die Steuereinrichtung dazu eingerichtet ist, mindestens ein trainiertes Maschinenlernverfahren und/oder Computer-Vision-Auswertungsverfahren bereitzustellen, dem mindestens einen trainierten Maschinenlernverfahren und/oder Computer-Vision-Auswertungsverfahren die erfasste linksseitige Abbildung und die erfasste rechtsseitige Abbildung als Eingangsdaten zuzuführen, wobei das mindestens eine trainierte Maschinenlernverfahren und/oder Computer-Vision-Auswertungsverfahren dazu eingerichtet und/oder trainiert ist, ausgehend von den erfassten Abbildungen ein Zielobjekt in den erfassten Abbildungen zu identifizieren und hierfür optimale Mikroskopparameter und/oder eine Änderung der Mikroskopparameter und/oder Steuerbefehle für die Aktorik zu schätzen und bereitzustellen, wobei die Steuereinrichtung ferner dazu eingerichtet ist, aus den geschätzten optimalen Mikroskopparametern und/oder der geschätzten Änderung der Mikroskopparameter Steuerbefehle für die Aktorik des Operationsmikroskops zu erzeugen und/oder die Aktorik des Operationsmikroskops entsprechend den erzeugten und/oder geschätzten Steuerbefehlen anzusteuern.

Ein Vorteil des Verfahrens und des Operationsmikroskops ist, dass eine Justage des Operationsmikroskops schneller durchgeführt werden kann. Das mindestens eine trainierte Maschinenlernverfahren und/oder Computer-Vision-Auswertungsverfahren schätzt die optimalen Mikroskopparameter und/oder die Änderung der Mikroskopparameter und/oder die Steuerbefehle insbesondere in einem einzigen Durchlauf, das heißt, es sind keine Zwischeniterationen notwendig. Insbesondere sind keine Optimierungsschleifen notwendig, sondern die optimalen Mikroskopparameter und/oder die Änderung der Mikroskopparameter und/oder die Steuerbefehle stehen nach einer einzigen Anwendung des mindestens einen trainierten Maschinenlernverfahrens und/oder Computer-Vision-Auswertungsverfahrens zur Verfügung. Insbesondere schätzt das mindestens eine trainierte Maschinenlernverfahren und/oder Computer-Vision-Auswertungsverfahren die optimalen Mikroskopparameter und/oder die Änderung der Mikroskopparameter und/oder die Steuerbefehle (zum Einstellen der optimalen Mikroskopparameter) ausgehend von einer (einzigen) erfassten linksseitigen Abbildung und einer (einzigen) erfassten rechtsseitigen Abbildung. Hierdurch können die optimalen Mikroskopparameter und/oder die Änderung der Mikroskopparameter und/oder die Steuerbefehle besonders schnell und zeitsparend geschätzt und bereitgestellt werden. Das Zielobjekt wird hierbei insbesondere ausschließlich durch das mindestens eine trainierte Maschinenlernverfahren und/oder Computer-Vision-Auswertungsverfahren identifiziert. Hierbei detektiert das mindestens eine trainierte Maschinenlernverfahren und/oder Computer-Vision-Auswertungsverfahren das Zielobjekt in der erfassten linkseitigen Abbildung und in der erfassten rechtsseitigen Abbildung. Insbesondere ist eine vorangehende Anwahl und/oder Auswahl und/oder Markierung des Zielobjektes nicht notwendig, sodass das Identifizieren und Schätzen insbesondere ohne eine vorangehende Anwahl und/oder Auswahl und/oder Markierung des Zielobjektes erfolgen. Auch eine Positionsvorgabe ist nicht notwendig, sodass das Identifizieren und Schätzen insbesondere ohne eine Positionsvorgabe oder Positionsmarkierung des Zielobjektes erfolgen. Bereits nach dem Erfassen einer (einzigen) linksseitigen Abbildung und einer (einzigen) rechtsseitigen Abbildung können mittels des mindestens einen trainierten Maschinenlernverfahrens und/oder Computer-Vision-Auswertungsverfahrens optimale Mikroskopparameter und/oder eine Änderung der Mikroskopparameter und/oder Steuerbefehle (zum Einstellen von optimalen Mikroskopparametern) bereitgestellt werden. Insbesondere erlauben es das Verfahren und das Operationsmikroskop, das Operationsmikroskop automatisiert in optimaler Weise, d.h. insbesondere in einer gewünschten und/oder bevorzugten Soll-Konfiguration, für ein chirurgisches Zielobjekt zu konfigurieren. Nach dem Durchführen des Verfahrens kann ein Chirurg direkt mit der Operation oder einem Zwischenschritt der Operation beginnen. Zur Erzeugung der Steuerbefehle ist es auf diese Weise insbesondere nicht notwendig, eine zeitliche Abfolge von Abbildungen oder eine Videosequenz aufzunehmen bzw. zu erfassen. Insbesondere kann das Vorliegen einer einzigen rechtseitigen oder linksseitigen Abbildung einer Stereoabbildung bereits ausreichend sein, um die Steuerbefehle zu erzeugen.

Das Operationsmikroskop ist insbesondere ein stereoskopisches Operationsmikroskop, bei dem ein linker und ein rechter Strahlengang ein stereoskopisches Erfassen des Erfassungsbereichs ermöglichen. Über den linken Strahlengang kann der Erfassungsbereich mittels der linken Kamera erfasst werden. Über den rechten Strahlengang kann der Erfassungsbereich mittels der rechten Kamera erfasst werden. Hierdurch können eine linke Abbildung und eine rechte Abbildung erfasst werden. Durch einen hierbei auftretenden Parallaxeneffekt lassen sich insbesondere auch Tiefeninformationen aus dem Erfassungsbereich gewinnen, welche insbesondere auch von dem mindestens einen trainierten Maschinenlernverfahren ausgewertet werden.

Die Aktorik des Operationsmikroskops umfasst insbesondere Aktoren und/oder Motoren. Mittels der Aktorik des Operationsmikroskops lässt sich insbesondere eine Position eines Mikroskopkopfes verändern, wobei der Mikroskopkopf insbesondere eine Abbildungsoptik des Operationsmikroskops umfasst. Ferner lassen sich mittels der Aktorik insbesondere Positionen von Linsen in der Abbildungsoptik des Operationsmikroskops verändern. Die Aktorik wird mittels der Steuereinrichtung gesteuert und/oder geregelt, wobei die Aktorik hierzu eine Sensorik zum Erfassen eines Ist-Zustands aufweisen kann.

Mikroskopparameter umfassen insbesondere eine Position des Mikroskopkopfes (z.B. in x-, y- und z-Richtung) sowie Parameter einer Abbildungsoptik (z.B. Vergrößerungsfaktor etc.). Insbesondere betreffen die Mikroskopparameter hierbei einen Fokus bzw. eine Fokusebene, eine Vergrößerung und/oder eine Zentrierung auf das Zielobjekt. Ein Fokus wird insbesondere durch Verändern eines Abstandes des Mikroskopkopfes zum Zielobjekt erreicht (insbesondere durch eine Bewegung in z-Richtung). Die Zentrierung erfolgt insbesondere durch Verändern einer Position des Mikroskopkopfes, wobei dieser hierbei insbesondere in einer Ebene parallel zu einer Objektebene verschoben wird (auch als xy-Ebene bezeichnet). Die Vergrößerung wird insbesondere durch Verändern einer Linsenposition von Linsen der Abbildungsoptik des Operationsmikroskops erreicht.

Eine Änderung der Mikroskopparameter bezeichnet insbesondere Differenzwerte für die Mikroskopparameter, die ausgehend von aktuellen Mikroskopparametern (Ist-Mikroskopparametern) zum Erreichen der optimalen Mikroskopparameter (Soll-Mikroskopparameter bzw. Soll-Konfiguration) eingestellt werden müssen.

Steuerbefehle der Aktorik bezeichnen insbesondere Steuerbefehle, die ausgehend von aktuellen Mikroskopparametern (Ist-Mikroskopparametern) zum Erreichen der optimalen Mikroskopparameter (Soll-Mikroskopparameter) eingestellt werden müssen.

Teile der Steuereinrichtung können einzeln oder zusammengefasst als eine Kombination von Hardware und Software ausgebildet sein, beispielsweise als Programmcode, der auf einem Mikrocontroller oder Mikroprozessor ausgeführt wird. Es kann jedoch auch vorgesehen sein, dass Teile einzeln oder zusammengefasst als anwendungsspezifische integrierte Schaltung (ASIC) oder feldprogrammierbares Gatterfeld (FPGA) ausgebildet sind. Insbesondere umfasst die Steuereinrichtung mindestens eine Recheneinrichtung und mindestens einen Speicher.

Das trainierte Maschinenlernverfahren wird oder wurde im Rahmen einer dem Verfahren vorangehenden Trainingsphase anhand mindestens eines Trainingsdatensatzes trainiert.

Ein solcher Trainingsdatensatz umfasst Paare aus jeweils einer rechten und einer linken Abbildung, welche zumindest mit optimalen Mikroskopparametern und/oder einer Änderung der Mikroskopparameter und/oder Steuerbefehlen für die Aktorik des Operationsmikroskops annotiert sind. Die Annotation bildet die Grundwahrheit beim Trainieren des mindestens einen Maschinenlernverfahrens. Es kann ferner vorgesehen sein, dass auch eine Art und eine Position des chirurgischen Zieles in den paarweisen Abbildungen als Annotation bzw. Grundwahrheit vorgegeben ist. Das Trainieren selbst erfolgt in an sich bekannter Weise, beispielsweise im Wege des überwachten Lernens. Die optimalen Mikroskopparameter werden insbesondere manuell auf Grundlage einer gewünschten optimalen Konfiguration des Operationsmikroskops bestimmt bzw. definiert. Anders ausgedrückt: Es wird insbesondere manuell definiert, mit welcher Konfiguration ein optimales Arbeiten bei einem chirurgischen Zielobjekt erfolgen kann oder soll. Die jeweilige Soll-Konfiguration wird als Grundwahrheit zum Annotieren der jeweils zugehörig paarweise erfassten Abbildungen verwendet.

In einer Alternative wird das Zielobjekt alternativ oder zusätzlich ausgehend von den erfassten Abbildungen mittels (mindestens) eines Computer-Vision-Auswertungsverfahrens in den erfassten Abbildungen identifiziert und es werden hierfür optimale Mikroskopparameter und/oder eine Änderung von Mikroskopparametern und/oder Steuerbefehle für die Aktorik des Operationsmikroskops geschätzt. Nachfolgend wird beispielhaft eine Ausführungsform des Computer-Vision-Auswertungsverfahrens anhand eines Augenlimbus als Zielobjekt beschrieben. Nachdem die linksseitige und die rechtsseitige Abbildung erfasst wurden, werden mehrere ringförmige Schablonen(-Abbildungen) signaltechnisch mit den erfassten Abbildungen korreliert. Hierbei erfolgt insbesondere eine örtliche Korrelation mit den erfassten Abbildungen. Die Schablonen(-Abbildungen) umfassen bzw. beinhalten hierbei jeweils Ringe unterschiedlicher Größe (bzw. unterschiedlichen Durchmessers). Ein großer Korrelationswert zwischen einer der Schablonen(-Abbildungen) und den erfassten Abbildungen bedeutet hierbei eine Übereinstimmung. Der größte Korrelationswert bestimmt hierbei eine Position und einen Radius des Limbus in den erfassten Abbildungen. Wenn der Korrelationswert größer als ein vorgegebener Schwellwert ist, wurde das (chirurgische) Zielobjekt, in diesem Beispiel der Limbus, erfolgreich erkannt. Anderenfalls konnte das (chirurgische) Zielobjekt, also der Limbus, in den erfassten Abbildungen nicht erkannt werden. Das Verfahren wird in diesem Fall insbesondere erfolglos beendet. Aus dem Ergebnis mit dem größten Korrelationswert kann dann die Mittelposition des Limbus in den beiden erfassten Abbildungen bestimmt werden (dies ist insbesondere der Mittelpunkt des jeweiligen Rings in der Schablone an der Position mit dem größten Korrelationswert). Ausgehend von dem derart identifizierten (chirurgischen) Zielobjekt, im Beispiel ausgehend von der Mittelposition des Limbus in den beiden erfassten Abbildungen, können dann optimale Mikroskopparameter und/oder eine Änderung von Mikroskopparametern und/oder Steuerbefehle für die Aktorik des Operationsmikroskops geschätzt werden. Beispielsweise kann die jeweils bestimmte Mittelposition in die Mitte des jeweiligen Erfassungsbereichs der Kameras gebracht werden. Das Auffinden des Augenlimbus in einer erfassten Abbildung des Auges ist beispielsweise in der WO 2009/080790 A1 beschrieben. Sollen andere (chirurgische) Zielobjekte identifiziert werden, so werden insbesondere entsprechend hierfür eingerichtete und/oder geeignete Schablonen(-Abbildungen) verwendet. Das Vorgehen ist aber für andere (chirurgische) Zielobjekte grundsätzlich analog.

In einer Ausführungsform ist vorgesehen, dass dem mindestens einen trainierten Maschinenlernverfahren zusätzlich auch während des Erfassens der Abbildungen eingestellte Mikroskopparameter als Eingangsdaten zugeführt werden. Hierdurch werden dem mindestens einen trainierten Maschinenlernverfahren insbesondere die Ist-Mikroskopparameter beim Erfassen der beiden Abbildungen zugeführt. Hierdurch kann eine Genauigkeit beim Schätzen der Mikroskopparameter und/oder der Änderung der Mikroskopparameter und/oder der Steuerbefehle verbessert werden. Entsprechend werden solche Ist-Mikroskopparameter auch bereits während der Trainingsphase des mindestens einen Maschinenlernverfahrens als jeweilige Annotation bzw. Grundwahrheit zu den Abbildungspaaren in den Trainingsdaten berücksichtigt. Das Berücksichtigen erlaubt es insbesondere, auch unterschiedliche Größenskalen von gleichartigen chirurgischen Zielobjekten, insbesondere in Unterscheidung zu unterschiedlichen Vergrößerungsstufen, verbessert handhaben zu können.

In einer Ausführungsform ist vorgesehen, dass eine Art einer Operation und/oder eine Phase der Operation erfasst und/oder empfangen werden und dem trainierten Maschinenlernverfahren als Eingangsdaten zugeführt werden. Hierdurch können das Identifizieren des chirurgischen Zielobjekts und das Schätzen der Mikroskopparameter und/oder der Änderung der Mikroskopparameter und/oder der Steuerbefehle im Hinblick auf eine Genauigkeit verbessert werden. Die Art der Operation und/oder die Phase der Operation können beispielsweise mittels einer Bedienschnittstelle von einem Nutzer abgefragt und/oder erfasst werden. Ebenso ist es möglich, die Art der Operation und/oder die Phase der Operation von einer anderen Quelle zu erhalten. Beispielsweise kann eine Datenbank diese Informationen bereitstellen und/oder diese Informationen können aus einem Operationsplan und/oder einem Operationsprotokoll abgerufen werden. Die Art der Operation und/oder die Phase der Operation werden dann auch bereits in der Trainingsphase als jeweils mit den Abbildungspaaren verknüpfte Grundwahrheit berücksichtigt.

In einer Ausführungsform ist vorgesehen, dass eine Art einer Operation und/oder eine Phase der Operation erfasst und/oder empfangen werden, wobei das mindestens eine trainierte Maschinenlernverfahren und/oder Computer-Vision-Auswertungsverfahren in Abhängigkeit der Art der Operation und/oder der Phase der Operation aus mehreren trainierten Maschinenlernverfahren und/oder Computer-Vision-Auswertungsverfahren ausgewählt wird, wobei das ausgewählte mindestens eine trainierte Maschinenlernverfahren und/oder Computer-Vision-Auswertungsverfahren verwendet wird. Hierdurch kann ein auf die Art der Operation und/oder die Phase der Operation spezialisiertes Maschinenlernverfahren und/oder Computer-Vision-Auswertungsverfahren ausgewählt und bereitgestellt werden. Dies ermöglicht es, eine Genauigkeit zu erhöhen. Ferner benötigt ein spezialisiertes Maschinenlernverfahren in der Regel bei der Anwendung weniger Rechenleistung, sodass Ressourcen eingespart werden können. Insbesondere kann ein spezialisiertes Maschinenlernverfahren in der Regel schneller ausgeführt werden. In einer Trainingsphase werden entsprechend mehrere auf die Art und/oder die Phase der Operationen spezialisierte Maschinenlernverfahren erzeugt bzw. entsprechend trainiert.

In einer entsprechenden Ausführungsform des Operationsmikroskops ist vorgesehen, dass das Operationsmikroskop mindestens eine Schnittstelle aufweist, wobei die mindestens eine Schnittstelle dazu eingerichtet ist, eine Art einer Operation und/oder eine Phase der Operation zu erfassen und/oder zu empfangen, wobei die Steuereinrichtung ferner dazu eingerichtet ist, die Art der Operation und/oder die Phase der Operation dem mindestens einen trainierten Maschinenlernverfahren als Eingangsdaten zuzuführen und/oder das mindestens eine trainierte Maschinenlernverfahren und/oder Computer-Vision-Auswertungsverfahren in Abhängigkeit der Art der Operation und/oder der Phase der Operation aus mehreren trainierten Maschinenlernverfahren und/oder Computer-Vision-Auswertungsverfahren auszuwählen und das ausgewählte mindestens eine trainierte Maschinenlernverfahren und/oder Computer-Vision-Auswertungsverfahren zum Verwenden bereitzustellen. Die mindestens eine Schnittstelle kann beispielsweise eine Nutzerschnittstelle in Form einer Anzeige- und Bedieneinrichtung sein. Alternativ oder zusätzlich kann die Schnittstelle auch zur Anbindung an ein Datenverarbeitungssystem dienen, das beispielsweise einen Operationsplan bereitstellt und ausgehend von dem Operationsplan der Schnittstelle die Art der Operation und/oder der Phase der Operation zuführt.

Erfindungsgemäß ist vorgesehen, dass das mindestens eine trainierte Maschinenlernverfahren und/oder Computer-Vision-Auswertungsverfahren zu jeder geschätzten Ausgabe einen Konfidenzwert schätzt und bereitstellt, wobei der bereitgestellte Konfidenzwert mit einem vorgegebenen Schwellenwert verglichen wird, wobei das Verfahren abgebrochen wird, wenn der bereitgestellte Konfidenzwert unterhalb des vorgegebenen Schwellenwertes liegt. Hierdurch kann sichergestellt werden, dass das Identifizieren des chirurgischen Zielobjektes und/oder das Schätzen der Mikroskopparameter und/oder der Änderung der Mikroskopparameter und/oder der Steuerbefehle eine vorgegebene Konfidenz aufweist, und die Mikroskopparameter und/oder die Änderung der Mikroskopparameter und/oder die Steuerbefehle nur bei Erreichen einer ausreichenden Konfidenz eingestellt bzw. verändert werden. Hierdurch kann insbesondere eine Fehlbedienung oder Fehlfunktion des Operationsmikroskops verhindert werden. Der Konfidenzwert und der Schwellenwert können auch in Form von Vektoren mit jeweils mehreren Einträgen vorliegen. Die Einträge werden dann einzeln miteinander verglichen.

In einer Ausführungsform ist vorgesehen, dass ein Startbefehl als Eingabe erfasst wird, wobei das Erfassen der Abbildungen, das Identifizieren und das Schätzen der optimalen Mikroskopparameter und/oder der Änderung der Mikroskopparameter und/oder der Steuerbefehle und das Ansteuern gestartet werden, wenn der Startbefehl erfasst wurde. Dies ermöglicht es, das Verfahren immer dann zu starten, wenn dies seitens eines Nutzers, insbesondere eines Chirurgen oder Assistenten, gewünscht ist.

In einer entsprechenden Ausführungsform des Operationsmikroskops weist das Operationsmikroskop eine Nutzerschnittstelle auf, wobei die Nutzerschnittstelle dazu eingerichtet ist, einen Startbefehl als Eingabe zu erfassen, wobei die Steuereinrichtung dazu eingerichtet ist, das Erfassen der Abbildungen und das Schätzen der optimalen Mikroskopparameter und/oder der Änderung der Mikroskopparameter und/oder der Steuerbefehle und das Ansteuern zu starten, wenn der Startbefehl erfasst wurde. Die Nutzerschnittstelle kann beispielsweise ein Bedienelement umfassen, beispielsweise einen Taster, mit dem das Verfahren ausgelöst werden kann. Insbesondere kann mittels der Nutzerschnittstelle, insbesondere mittels des Tasters, verfahrensgemäß ein automatisiertes Einstellen einer Vergrößerung, eines Fokus und/oder einer Zentrierung auf das chirurgische Zielobjekt gestartet werden.

In einer Ausführungsform ist vorgesehen, dass das mindestens eine trainierte Maschinenlernverfahren mindestens ein trainiertes Neuronales Netz umfasst. Das trainierte Neuronale Netz kann insbesondere ein trainiertes Faltungsnetz (engl. Convolutional Neural Network, CNN) sein. Grundsätzlich können jedoch auch andere Maschinenlernverfahren eingesetzt werden.

Weitere Merkmale zur Ausgestaltung des Operationsmikroskops ergeben sich aus der Beschreibung von Ausgestaltungen des Verfahrens. Die Vorteile des Operationsmikroskops sind hierbei jeweils die gleichen wie bei den Ausgestaltungen des Verfahrens.

Nachfolgend wird die Erfindung anhand bevorzugter Ausführungsbeispiele unter Bezugnahme auf die Figuren näher erläutert. Hierbei zeigen:
- Fig. 1: eine schematische Darstellung einer Ausführungsform des Operationsmikroskops;
- Fig. 2: ein schematisches Ablaufdiagramm zur Verdeutlichung einer Trainingsphase des Maschinenlernverfahrens;
- Fig. 3: ein schematisches Ablaufdiagramm einer Ausführungsform des Verfahrens zum Betreiben eines Operationsmikroskops.

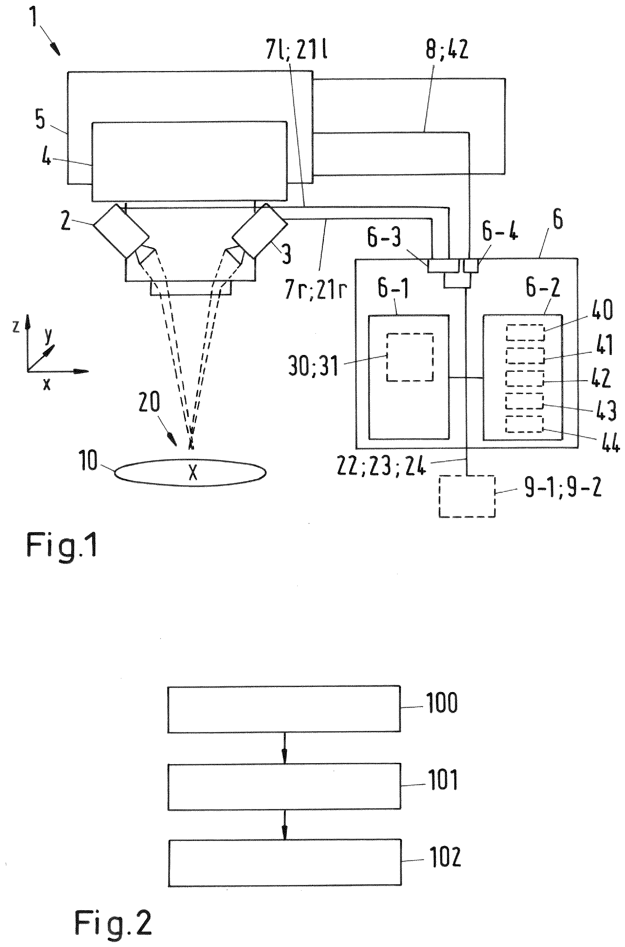

In Fig. 1 ist eine schematische Darstellung einer Ausführungsform des Operationsmikroskops 1 gezeigt. Das Operationsmikroskop 1 umfasst eine linkseitige Kamera 2, eine rechtsseitige Kamera 3 und eine in einem Mikroskopkopf 4 angeordnete Aktorik 5. Die linksseitige Kamera 2 und die rechtsseitige Kamera 3 sind an einem Strahlengang einer stereoskopischen Abbildungsoptik angeordnet. Die Aktorik 5 umfasst beispielsweise Aktoren und Schrittmotoren. Ferner umfasst das Operationsmikroskop 1 eine Steuereinrichtung 6. Die Steuereinrichtung 6 umfasst eine Recheneinrichtung 6-1 und einen Speicher 6-2. Die Recheneinrichtung 6-1 umfasst mindestens einen Mikroprozessor. Die Recheneinrichtung 6-1 kann auf in dem Speicher 6-2 gespeicherte Daten zugreifen und Rechenoperationen auf den Daten ausführen. Insbesondere führt die Recheneinrichtung 6-1 einen in dem Speicher 6-2 hinterlegten Programmcode aus, um Maßnahmen des in dieser Offenbarung beschriebenen Verfahrens durchzuführen. Die Steuereinrichtung 6 umfasst ferner Schnittstellen 6-3, 6-4, die über Signalleitungen 7l, 7r, 8 mit den Kameras 2, 3 und der Aktorik 5 verbunden sind.

Nachfolgend wird eine Ausführungsform des Verfahrens zum Betreiben eines Operationsmikroskops 1 anhand des Operationsmikroskops 1 erläutert.

In einem Erfassungsbereich 10 des Operationsmikroskops 1 ist ein chirurgisches Zielobjekt 20 angeordnet. Dies kann beispielsweise ein Limbus des Auges oder Gliome bei der Kraniotomie etc. sein.

Mittels der linksseitigen Kamera 2 wird eine linksseitige Abbildung 211 des Erfassungsbereichs 10 erfasst. Mittels der rechtsseitigen Kamera 3 wird, insbesondere zeitgleich, eine rechtsseitige Abbildung 21r des Erfassungsbereichs 10 erfasst. Die Abbildungen 211, 21r bilden zusammen insbesondere eine stereoskopische Abbildung. Die erfassten Abbildungen 21l, 21r werden der Steuereinrichtung 6 über die Schnittstelle 6-3 zugeführt.

Die Steuereinrichtung 6 stellt ein trainiertes Maschinenlernverfahren 30 bereit. Hierzu ist eine Beschreibung des trainierten Maschinenlernverfahrens 30 in dem Speicher 6-2 hinterlegt und wird von der Recheneinrichtung 6-1 zum Bereitstellen ausgeführt. Insbesondere umfasst das trainierte Maschinenlernverfahren 30 ein trainiertes Neuronales Netz 31, insbesondere ein Faltungsnetz. In dem Speicher 6-2 sind eine Strukturbeschreibung und Parameter (Filterparameter, Gewichtungen etc.) des Neuronalen Netzes 31 hinterlegt. Die Recheneinrichtung 6-2 stellt das trainierte Neuronale Netz 31 gemäß der Strukturbeschreibung und den Parametern bereit bzw. führt dieses entsprechend aus.

Die erfasste linkseitige Abbildung 21l und die erfasste rechtsseitige Abbildung 21r werden dem bereitgestellten trainierten Maschinenlernverfahren 30, insbesondere dem trainierten Neuronalen Netz 31, als Eingangsdaten zugeführt.

Mittels des trainierten Maschinenlernverfahrens 30, insbesondere mittels des trainierten Neuronalen Netzes 31, werden ausgehend von den erfassten Abbildungen 211, 21r ein Zielobjekt 20 in den erfassten Abbildungen 211, 21r identifiziert und hierfür optimale Mikroskopparameter 40 und/oder eine Änderung 41 der Mikroskopparameter 40 und/oder Steuerbefehle 42 für die Aktorik 5 des Operationsmikroskops 1 geschätzt.

Ob optimale Mikroskopparameter 40 und/oder eine Änderung 41 von Mikroskopparametern 40 oder ob Steuerbefehle 42 von dem trainierten Maschinenlernverfahren 30, insbesondere dem trainierten Neuronalen Netz 31, geschätzt werden, hängt hierbei davon ab, wie das trainierte Maschinenlernverfahren 30 ausgestaltet ist, das heißt, wie eine Struktur des mindestens einen Maschinenlernverfahrens 30 ist und worauf es in einer Trainingsphase trainiert wurde.

Wurden optimale Mikroskopparameter 40 und/oder eine Änderung 41 von Mikroskopparametern 40 geschätzt, so werden mittels der Steuereinrichtung 6 aus den geschätzten optimalen Mikroskopparametern 40 und/oder der geschätzten Änderung 41 der Mikroskopparameter 40 Steuerbefehle für eine Aktorik 5 des Operationsmikroskops 1 erzeugt. Die Steuereinrichtung 6 steuert die Aktorik 5 gemäß den erzeugten Steuerbefehlen 42 an.

Wurden Steuerbefehle 42 für die Aktorik 4 geschätzt, so steuert die Steuereinrichtung 6 die Aktorik 4 gemäß den geschätzten Steuerbefehlen 42 an.

Die optimalen Mikroskopparameter 40 umfassen insbesondere Parameter für einen Fokus, eine Vergrößerung und eine Zentrierung des Mikroskopkopfes 5. Die Parameter für den Fokus werden insbesondere durch Bewegen des Mikroskopkopfes 4 in z-Richtung eingestellt. Parameter der Vergrößerung werden insbesondere durch Verändern einer Position von Linsen einer Abbildungsoptik im Mikroskopkopf 4 eingestellt. Parameter zur Zentrierung des Mikroskopkopfes 4 werden insbesondere durch Verfahren des Mikroskopkopfes 4 in x-y-Richtung eingestellt. Grundsätzlich können die optimalen Mikroskopparameter 40 aber auch andere Parameter des Operationsmikroskops 1 umfassen, z.B. Parameter einer Lichtquelle des Operationsmikroskops und/oder Kameraparameter etc.

Alternativ oder zusätzlich wird das Zielobjekt 20 ausgehend von den erfassten Abbildungen 21l, 21r mittels (mindestens) eines Computer-Vision-Auswertungsverfahrens in den erfassten Abbildungen 21l, 21r identifiziert und es werden hierfür optimale Mikroskopparameter 40 und/oder eine Änderung 41 von Mikroskopparametern 40 und/oder Steuerbefehle 42 für die Aktorik 5 des Operationsmikroskops 1 geschätzt. Das Vorgehen wurde hierbei beispielhaft bereits in der allgemeinen Beschreibung erläutert.

Es kann vorgesehen sein, dass dem trainierten Maschinenlernverfahren 30, insbesondere dem trainierten Neuronalen Netz 31, zusätzlich auch während des Erfassens der Abbildungen 21l, 21r eingestellte Mikroskopparameter 43 als Eingangsdaten zugeführt werden. Diese Mikroskopparameter 43 können auch als Ist-Mikroskopparameter bezeichnet werden, da diese einen Ist-Zustand einer Konfiguration des Operationsmikroskops 1 beim Erfassen der Abbildungen 21l, 21r beschreiben. Diese Ist-Mikroskopparameter 43 umfassen insbesondere Parameter für einen Fokus, eine Vergrößerung und/oder eine Zentrierung des Operationsmikroskops 1.

Es kann vorgesehen sein, dass eine Art 22 einer Operation und/oder eine Phase 23 der Operation erfasst und/oder empfangen werden und dem trainierten Maschinenlernverfahren 30, insbesondere dem trainierten Neuronalen Netz 31, als Eingangsdaten zugeführt werden. Hierzu weist das Operationsmikroskop 1 eine Schnittstelle 9-1 auf, mit der die Art 22 der Operation und/oder die Phase 23 der Operation erfasst und/oder empfangen werden kann. Zum Erfassen ist die Schnittstelle 9-1 beispielsweise als Nutzerschnittstelle 9-2 ausgestaltet, sodass ein Nutzer, insbesondere ein Chirurg oder ein Assistent, die Art 22 und/oder die Phase 23 der Operation eingeben können. Alternativ oder zusätzlich kann die Art 22 und/oder die Phase 23 der Operation auch von einem Operationsplanungssystem abgefragt und/oder bereitgestellt werden, welches beispielsweise einen Operationsplan bereitstellt.

Es kann auch vorgesehen sein, dass die Art 22 der Operation und/oder die Phase 23 der Operation erfasst und/oder empfangen werden, wobei das trainierte Maschinenlernverfahren 30, insbesondere das trainierte Neuronale Netz 31, und/oder das Computer-Vision-Auswertungsverfahren in Abhängigkeit der Art 22 der Operation und/oder der Phase 23 der Operation aus mehreren trainierten

Maschinenlernverfahren 30, insbesondere mehreren trainierten Neuronalen Netzen 31, und/oder Computer-Vision-Auswertungsverfahren ausgewählt wird, wobei das ausgewählte trainierte Maschinenlernverfahren 30, insbesondere das ausgewählte trainierte Neuronale Netz 31, und/oder das ausgewählte Computer-Vision-Auswertungsverfahren verwendet wird.

Erfindungsgemäß ist vorgesehen, dass das trainierte Maschinenlernverfahren 30, insbesondere das trainierte Neuronale Netz 31, und/oder das Computer-Vision-Auswertungsverfahren zu jeder geschätzten Ausgabe einen Konfidenzwert schätzt und bereitstellt, wobei der bereitgestellte Konfidenzwert mit einem vorgegebenen Schwellenwert 44 verglichen wird, wobei das Verfahren abgebrochen wird, wenn der bereitgestellte Konfidenzwert unterhalb des vorgegebenen Schwellenwertes 44 liegt. Eine Ausgabe des trainierten Maschinenlernverfahrens 30, insbesondere des trainierten Neuronalen Netzes 31, und/oder des Computer-Vision-Auswertungsverfahrens umfasst hierbei sowohl ein geschätztes chirurgisches Zielobjekt 20 als auch optimale Mikroskopparameter 40 und/oder eine Änderung 41 der Mikroskopparameter 40 und/oder Steuerbefehle 42 für die Aktorik 5. Insbesondere wird für die jeweiligen Ausgabewerte jeweils ein Konfidenzwert der Schätzung bereitgestellt und mit dem vorgegebenen Schwellenwert 44 verglichen. Entsprechend können der Konfidenzwert und der vorgegebene Schwellenwert 44 auch als Vektoren mit mehreren Einträgen gemäß den jeweiligen Ausgabewerten ausgestaltet sein. Hierdurch wird eine Konfiguration des Operationsmikroskops 1 nur verändert, wenn ein Mindestmaß an Sicherheit beim Identifizieren des chirurgischen Zielobjekts 20 und/oder beim Schätzen erreicht ist. Anderenfalls erfolgt keine Änderung der Konfiguration des Operationsmikroskops.

Es kann vorgesehen sein, dass ein Startbefehl 24 als Eingabe erfasst wird, wobei das Erfassen der Abbildungen 21l, 21r, das Identifizieren und das Schätzen der optimalen Mikroskopparameter 40 und/oder der Änderung 41 der Mikroskopparameter 40 und/oder der Steuerbefehle 42 und das Ansteuern gestartet werden, wenn der Startbefehl 24 erfasst wurde. Hierzu weist das Operationsmikroskop 1 eine Nutzerschnittstelle 9-2 auf. Diese kann beispielsweise ein Bedienelement in Form eines Tasters umfassen, mit dem der Startbefehl 24 zum Starten der Maßnahmen des Verfahrens durch einen Nutzer, insbesondere durch einen Chirurgen oder einen Assistenten, gegeben werden kann.

In der Fig. 2 ist ein schematisches Ablaufgramm zur Verdeutlichung eines Ablaufs einer Trainingsphase des mindestens einen Maschinenlernverfahrens, insbesondere des trainierten mindestens einen Neuronalen Netzes, gezeigt.

In einer Maßnahme 100 wird ein Trainingsdatensatz zusammengestellt. Hierbei werden paarweise linksseitig und rechtsseitig erfasste Abbildungen (Stereoabbildungen) mit Annotationen versehen, das heißt mit einer Grundwahrheit verknüpft. Die Abbildungen zeigen ein chirurgisches Zielobjekt (z.B. ein Zentrum eines Limbus des Auges, Gliome bei der Kraniotomie etc.). Die Annotationen umfassen je nach Ausgestaltung optimale Mikroskopparameter (z.B. als optimale x-y-z-Positionen eines Mikroskopkopfes und Linsenpositionen bzw. eine Vergrößerungsangabe einer Abbildungsoptik: 7,5x usw.) und/oder eine Änderung der Mikroskopparameter (z.B. in Form von Differenzwerten; z.B. als x-y-z-Positionsdifferenz: 200 µm in x-Richtung, -400 µm in y-Richtung usw.) und/oder Steuerbefehle für eine Aktorik des Operationsmikroskops. Die optimalen Mikroskopparameter werden insbesondere manuell auf Grundlage einer gewünschten optimalen Konfiguration des Operationsmikroskops bestimmt. Anders ausgedrückt: Es wird insbesondere manuell definiert, mit welcher Konfiguration ein optimales Arbeiten bei einem chirurgischen Zielobjekt erfolgen kann oder soll. Die jeweilige Soll-Konfiguration wird als Grundwahrheit zum Annotieren der jeweils zugehörig paarweise erfassten Abbildungen verwendet.

Es kann ferner vorgesehen sein, dass der Trainingsdatensatz weitere Daten umfasst. Hierdurch kann insbesondere eine Genauigkeit beim Schätzen erhöht werden. Die weiteren Daten können insbesondere Mikroskopparameter sein, die einen Ist-Zustand bzw. eine Ist-Konfiguration des Operationsmikroskops beim Erfassen der jeweiligen paarweisen Abbildungen (Stereoabbildungen) beschreibt. Insbesondere sind dies ein Ist-Zustand eines Fokus (z.B. in Form einer z-Position, vgl. Fig. 1), einer Vergrößerung (z.B. 3,2x etc.) und einer Zentrierung bzw. einer Position eines Mikroskopkopfes (z.B. eine x-y-Position eines Mikroskopkopfes). Ferner können die weiteren Daten auch Eigenschaften des Operationsmikroskops umfassen (beispielsweise eine Art der Objektivlinse, z.B. 200f etc.).

In einer Maßnahme 101 werden die paarweise bereitgestellten Abbildungen dem zu trainierenden Maschinenlernverfahren als Eingangsdaten zugeführt. Eine geschätzte Ausgabe des Maschinenlernverfahrens wird anschließend mit der Grundwahrheit verglichen, um ausgehend von einem Vergleichsergebnis Parameter des Maschinenlernverfahrens anzupassen. Dies erfolgt für den gesamten Trainingsdatensatz in mehreren Iterationen in an sich bekannter Weise, beispielsweise im Wege des überwachten Lernens. Das Training wird solange fortgeführt, bis eine vorgegebene funktionale Güte beim Schätzen erreicht wird. Der Trainingsdatensatz kann hierzu auch in zwei Teile aufgeteilt werden, wobei der eine Teil zum Trainieren und der andere Teil zum Testen der funktionalen Güte verwendet wird.

In einer Maßnahme 102 wird das trainierte Maschinenlernverfahren, insbesondere in Form einer Strukturbeschreibung und von Parametern (Filterparameter, Gewichtungen etc.), in einen Speicher der Steuereinrichtung des Operationsmikroskops eingeladen. Die Steuereinrichtung kann das trainierte Maschinenlernverfahren dann anwenden und dessen Funktionalität bereitstellen.

Es kann vorgesehen sein, dass ein Maschinenlernverfahren für mehrere chirurgische Zielobjekte trainiert und verwendet wird. Es kann jedoch auch vorgesehen sein, speziell trainierte Maschinenlernverfahren in Abhängigkeit einer Art und/oder einer Phase einer Operation zu trainieren und zur jeweiligen Anwendung auszuwählen und bereitzustellen. Hierdurch kann eine benötigte Rechenleistung beim Anwenden des jeweils spezialisierten Maschinenlernverfahrens reduziert werden.

Das Maschinenlernverfahren umfasst insbesondere mindestens ein Neuronales Netz, insbesondere mindestens ein Faltungsnetz.

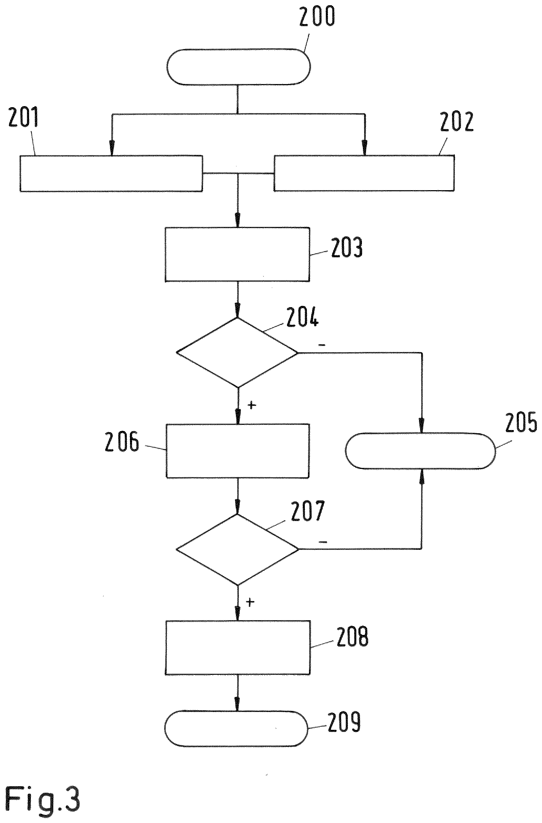

In der Fig. 3 ist ein schematisches Ablaufdiagramm einer Ausführungsform des Verfahrens zum Betreiben eines Operationsmikroskops gezeigt. Die Maßnahmen des Verfahrens werden gestartet 200, sobald ein Startbefehl erfasst wurde, beispielsweise an einer Nutzerschnittstelle des Operationsmikroskops.

Nach dem Starten der Maßnahmen wird in den Maßnahmen 201, 202 zeitgleich eine linksseitige Abbildung eines Erfassungsbereichs mittels einer linksseitigen Kamera des Operationsmikroskops und eine rechtsseitige Abbildung eines Erfassungsbereichs mittels einer rechtsseitigen Kamera des Operationsmikroskops erfasst.

In einer Maßnahme 203 wird mittels mindestens eines trainierten Maschinenlernverfahrens, insbesondere mittels mindestens eines trainierten Neuronalen Netzes, ausgehend von den erfassten Abbildungen ein (chirurgisches) Zielobjekt in den erfassten Abbildungen identifiziert. Bei dem chirurgischen Zielobjekt kann es sich beispielsweise um einen Limbus eines Auges oder Gliome handeln.

In einer Maßnahme 204 wird überprüft, ob eine Konfidenz beim Identifizieren des chirurgischen Zielobjekts einen vorgegebenen Schwellenwert überschreitet. Ein Konfidenzwert wird hierbei insbesondere von dem mindestens einen trainierten Maschinenlernverfahren für jede Ausgabe geschätzt und bereitgestellt. Beispielsweise werden für mehrere mögliche chirurgische Zielobjekte jeweils Wahrscheinlichkeiten dafür geschätzt, dass (an einer geschätzten Position in den Abbildungen) ein chirurgisches Zielobjekt einer bestimmten Art vorliegt, wobei zu jeder der Wahrscheinlichkeiten ein Konfidenzwert geschätzt wird. In der Regel wird dasjenige chirurgische Zielobjekt aus dem Ergebnis ausgewählt, für das der höchste Wahrscheinlichkeitswert (als Konfidenz) angegeben wird. Dieser Wahrscheinlichkeitswert bzw. die geschätzte Konfidenz wird dann mit dem vorgegebenen Schwellenwert verglichen.

Erreicht die geschätzte Konfidenz nicht den vorgegebenen Schwellenwert, so wird das Verfahren abgebrochen und in einer Maßnahme 205 erfolglos beendet. Es kann hierbei vorgesehen sein, dass eine entsprechende Fehlermeldung ausgegeben wird, beispielsweise auf einer Anzeigeeinrichtung des Operationsmikroskops.

Erreicht oder überschreitet die geschätzte Konfidenz hingegen den vorgegebenen Schwellenwert, so werden in einer Maßnahme 206 mittels des mindestens einen trainierten Maschinenlernverfahrens, insbesondere mittels des mindestens einen trainierten Neuronalen Netzes, optimale Mikroskopparameter und/oder eine Änderung der Mikroskopparameter und/oder Steuerbefehle für eine Aktorik des Operationsmikroskops geschätzt.

Die Maßnahmen 203 und 206 können mittels eines (einzigen) trainierten Maschinenlernverfahrens durchgeführt werden. Es kann jedoch auch vorgesehen sein, dass die Maßnahmen 203 und 206 durch zwei getrennte trainierte Maschinenlernverfahren ausgeführt werden.

In einer Maßnahme 207 wird überprüft, ob die geschätzten optimalen Mikroskopparameter und/oder die Änderung der Mikroskopparameter und/oder die Steuerbefehle eingestellt werden können. Es kann beispielsweise der Fall auftreten, dass die hiermit angestrebte Soll-Konfiguration des Operationsmikroskops außerhalb eines möglichen Parameterbereichs von Aktoren etc. liegt, beispielsweise, weil eine für eine optimale Zentrierung notwenige Bewegung des Mikroskopkopfes außerhalb des möglichen Bewegungsbereichs liegt.

Kann die Soll-Konfiguration nicht eingestellt werden, so wird das Verfahren abgebrochen und in einer Maßnahme 205 erfolglos beendet. Es kann hierbei vorgesehen sein, dass eine entsprechende Fehlermeldung ausgegeben wird, beispielsweise auf einer Anzeigeeinrichtung des Operationsmikroskops.

Kann die Soll-Konfiguration hingegen eingestellt werden, so wird die Soll-Konfiguration in einer Maßnahme 208 eingestellt. Hierzu werden, je nachdem, was von dem mindestens einen Maschinenlernverfahren, insbesondere von dem mindestens einen Neuronalen Netz, geschätzt wurde, aus den geschätzten Mikroskopparametern und/oder der geschätzten Änderung der Mikroskopparameter Steuerbefehle für eine Aktorik des Operationsmikroskops erzeugt und an die Aktorik übermittelt. Wurden von dem mindestens einen trainierten Maschinenlernverfahren, insbesondere von dem trainierten mindestens einen Neuronalen Netz, hingegen Steuerbefehle geschätzt, so werden die geschätzten Steuerbefehle der Aktorik zugeführt.

Ist die Soll-Konfiguration eingestellt, so wird das Verfahren in einer Maßnahme 209 erfolgreich beendet. Es kann hierbei vorgesehen sein, dass eine entsprechende Erfolgsmeldung ausgegeben wird, beispielsweise auf einer Anzeigeeinrichtung des Operationsmikroskops.

Das Verfahren und das Operationsmikroskop ermöglichen das automatisierte Konfigurieren des Operationsmikroskops auf für ein erkanntes chirurgisches Zielobjekt optimale Mikroskopparameter. Ein Arbeitsfluss beim Operieren kann hierdurch verbessert werden.

### Bezugszeichenliste

- 1: Operationsmikroskop
- 2: linksseitige Kamera
- 3: rechtsseitige Kamera
- 4: Mikroskopkopf
- 5: Aktorik
- 6: Steuereinrichtung
- 6-1: Recheneinrichtung
- 6-2: Speicher
- 6-3: Schnittstelle
- 6-4: Schnittstelle
- 7l: Signalleitung (linksseitige Kamera)
- 7r: Signalleitung (rechtsseitige Kamera)
- 8: Signalleitung (Aktorik)
- 9-1: Schnittstelle
- 9-2: Nutzerschnittstelle
- 10: Erfassungsbereich
- 20: (chirurgisches) Zielobjekt
- 21l: linksseitige Abbildung
- 21r: rechtsseitige Abbildung
- 22: Art der Operation
- 23: Phase der Operation
- 24: Startbefehl
- 30: trainiertes Maschinenlernverfahren
- 31: trainiertes Neuronales Netz
- 40: optimale Mikroskopparameter
- 41: Änderung der Mikroskopparameter
- 42: Steuerbefehle
- 43: eingestellte Mikroskopparameter
- 44: Schwellenwert (für Konfidenz)
- 100-102: Maßnahmen (Trainingsphase)
- 200-209: Maßnahmen des Verfahrens

## Patentansprüche

1. Verfahren zum Betreiben eines Operationsmikroskops (1),
wobei eine linksseitige Abbildung (211) eines Erfassungsbereichs (10) des Operationsmikroskops (1) mittels einer linksseitigen Kamera (2) des Operationsmikroskops (1) erfasst wird,
wobei eine rechtsseitige Abbildung (21r) des Erfassungsbereichs (10) mittels einer rechtsseitigen Kamera (3) des Operationsmikroskops (1) erfasst wird,
wobei die erfasste linksseitige Abbildung (211) und die erfasste rechtsseitige Abbildung (21r) mindestens einem mittels einer Steuereinrichtung (6) des Operationsmikroskops (1) bereitgestellten trainierten Maschinenlernverfahren (30) und/oder Computer-Vision-Auswertungsverfahren als Eingangsdaten zugeführt werden, und
wobei mittels des mindestens einen trainierten Maschinenlernverfahrens (30) und/oder Computer-Vision-Auswertungsverfahrens ausgehend von den erfassten Abbildungen (21l,21r) ein chirurgisches Zielobjekt (20) in den erfassten Abbildungen (21l,21r) identifiziert wird, wobei beim Identifizieren des Zielobjektes (20) eine Art des Zielobjektes und eine Position des Zielobjektes (20) in den Abbildungen (21l,21r) bestimmt wird, und wobei für das identifizierte Zielobjekt (20) optimale Mikroskopparameter (40) und/oder eine Änderung (41) von Mikroskopparametern (40) und/oder Steuerbefehle (42) für eine Aktorik (5) des Operationsmikroskops (1) geschätzt werden,
wobei mittels der Steuereinrichtung (6) aus den geschätzten optimalen Mikroskopparametern (40) und/oder der geschätzten Änderung (41) der Mikroskopparameter (41) Steuerbefehle (42) für die Aktorik (5) des Operationsmikroskops (1) erzeugt werden und/oder wobei die Aktorik (5) entsprechend den erzeugten und/oder geschätzten Steuerbefehlen (42) angesteuert wird, sodass die Mikroskopparameter des Operationsmikroskops optimal auf das identifizierte chirurgische Zielobjekt konfiguriert sind,
wobei das mindestens eine trainierte Maschinenlernverfahren (30) und/oder Computer-Vision-Auswertungsverfahren zu jeder geschätzten Ausgabe einen Konfidenzwert schätzt und bereitstellt, wobei die Ausgabe das identifizierte chirurgische Zielobjekt (20) umfasst, wobei der bereitgestellte Konfidenzwert mit einem vorgegebenen Schwellenwert (44) verglichen wird, wobei das Verfahren abgebrochen wird, wenn der bereitgestellte Konfidenzwert unterhalb des vorgegebenen Schwellenwertes (44) liegt.

2. Verfahren nach Anspruch 1, **dadurch gekennzeichnet, dass** dem mindestens einen trainierten Maschinenlernverfahren (30) zusätzlich auch während des Erfassens der Abbildungen (21l,21r) eingestellte Mikroskopparameter (43) als Eingangsdaten zugeführt werden.

3. Verfahren nach einem der vorangegangenen Ansprüche, **dadurch gekennzeichnet, dass** eine Art (22) einer Operation und/oder eine Phase (23) der Operation erfasst und/oder empfangen werden und dem trainierten Maschinenlernverfahren (30) als Eingangsdaten zugeführt werden.

4. Verfahren nach einem der vorangegangenen Ansprüche, **dadurch gekennzeichnet, dass** eine Art (22) einer Operation und/oder eine Phase (23) der Operation erfasst und/oder empfangen werden, wobei das mindestens eine trainierte Maschinenlernverfahren (30) und/oder Computer-Vision-Auswertungsverfahren in Abhängigkeit der Art (22) der Operation und/oder der Phase (23) der Operation aus mehreren trainierten Maschinenlernverfahren (30) und/oder Computer-Vision-Auswertungsverfahren ausgewählt wird, wobei das ausgewählte mindestens eine trainierte Maschinenlernverfahren (30) und/oder Computer-Vision-Auswertungsverfahren verwendet wird.

5. Verfahren nach einem der vorangegangenen Ansprüche, **dadurch gekennzeichnet, dass** ein Startbefehl (24) als Eingabe erfasst wird, wobei das Erfassen der Abbildungen (21l,21r), das Identifizieren und das Schätzen der optimalen Mikroskopparameter (40) und/oder der Änderung (41) der Mikroskopparameter (40) und/oder der Steuerbefehle (42) und das Ansteuern gestartet werden, wenn der Startbefehl (24) erfasst wurde.

6. Verfahren nach einem der vorangegangenen Ansprüche, **dadurch gekennzeichnet, dass** das mindestens eine trainierte Maschinenlernverfahren (30) mindestens ein trainiertes Neuronales Netz (31) umfasst.

7. Verfahren nach einem der vorangegangenen Ansprüche, **dadurch gekennzeichnet, dass** das Identifizieren des chirurgischen Zielobjekts (20) mittels des mindestens einen trainierten Maschinenlernverfahrens (30) und/oder Computer-Vision-Auswertungsverfahrens und das Schätzen der optimalen Mikroskopparameter (40) und/oder der Änderung (41) von Mikroskopparametern (40) und/oder der Steuerbefehle (42) für die Aktorik (5) ausgehend von einer einzigen erfassten linkseitigen Abbildung (211) und einer einzigen erfassten rechtsseitigen Abbildung (21r) erfolgt.

8. Operationsmikroskop (1), umfassend:
eine linksseitige Kamera (2), eingerichtet zum Erfassen einer linkseitigen Abbildung (211) eines Erfassungsbereichs (10) des Operationsmikroskops (1), eine rechtsseitige Kamera (3), eingerichtet zum Erfassen einer rechtsseitigen Abbildung (21r) des Erfassungsbereichs (10),
eine Aktorik (5), eingerichtet zum Einstellen einer Konfiguration des Operationsmikroskops (1) gemäß Mikroskopparametern (40), und
eine Steuereinrichtung (6), wobei die Steuereinrichtung (6) dazu eingerichtet ist, mindestens ein trainiertes Maschinenlernverfahren (30) und/oder Computer-Vision-Auswertungsverfahren bereitzustellen, dem mindestens einen trainierten Maschinenlernverfahren (30) und/oder Computer-Vision-Auswertungsverfahren die erfasste linksseitige Abbildung (211) und die erfasste rechtsseitige Abbildung (21r) als Eingangsdaten zuzuführen,
wobei das mindestens eine trainierte Maschinenlernverfahren (30) und/oder Computer-Vision-Auswertungsverfahren dazu eingerichtet und/oder trainiert ist, ausgehend von den erfassten Abbildungen (211,211) ein chirurgisches Zielobjekt (20) in den erfassten Abbildungen (21l,21r) zu identifizieren, und beim Identifizieren des Zielobjektes eine Art des Zielobjektes (20) und eine Position des Zielobjektes (20) in den Abbildungen (21l,21r) zu bestimmen, und für das identifizierte Zielobjekt (20) optimale Mikroskopparameter (40) und/oder eine Änderung (41) der Mikroskopparameter (40) und/oder Steuerbefehle (42) für die Aktorik (5) zu schätzen und bereitzustellen,
wobei die Steuereinrichtung (6) ferner dazu eingerichtet ist, aus den geschätzten optimalen Mikroskopparametern (40) und/oder der geschätzten Änderung (41) der Mikroskopparameter (40) Steuerbefehle (42) für die Aktorik (5) des Operationsmikroskops (1) zu erzeugen und/oder die Aktorik (5) des Operationsmikroskops (1) entsprechend den erzeugten und/oder geschätzten Steuerbefehlen (42) anzusteuern, sodass die Mikroskopparameter des Operationsmikroskops optimal auf das identifizierte chirurgische Zielobjekt konfiguriert sind,
wobei das mindestens eine trainierte Maschinenlernverfahren (30) und/oder Computer-Vision-Auswertungsverfahren zu jeder geschätzten Ausgabe einen Konfidenzwert schätzt und bereitstellt, wobei die Ausgabe das identifizierte chirurgische Zielobjekt (20) umfasst,
wobei die Steuereinrichtung (6) ferner dazu eingerichtet ist, den bereitgestellten Konfidenzwert mit einem vorgegebenen Schwellenwert (44) zu vergleichen und das Verfahren abzubrechen, wenn der bereitgestellte Konfidenzwert unterhalb des vorgegebenen Schwellenwertes (44) liegt.

9. Operationsmikroskop (1) nach Anspruch 8, **gekennzeichnet durch** mindestens eine Schnittstelle (9-1,9-2), wobei die mindestens eine Schnittstelle (9-1,9-2) dazu eingerichtet ist, eine Art (22) einer Operation und/oder eine Phase (23) der Operation zu erfassen und/oder zu empfangen, wobei die Steuereinrichtung (6) ferner dazu eingerichtet ist, die Art (22) der Operation und/oder die Phase (23) der Operation dem mindestens einen trainierten Maschinenlernverfahren (30) als Eingangsdaten zuzuführen und/oder das mindestens eine trainierte Maschinenlernverfahren (30) und/oder Computer-Vision-Auswertungsverfahren in Abhängigkeit der Art (22) der Operation und/oder der Phase (23) der Operation aus mehreren trainierten Maschinenlernverfahren (30) und/oder Computer-Vision-Auswertungsverfahren auszuwählen und das ausgewählte mindestens eine trainierte Maschinenlernverfahren (30) und/oder Computer-Vision-Auswertungsverfahren zum Verwenden bereitzustellen.

10. Operationsmikroskop (1) nach Anspruch 8 oder 9, **gekennzeichnet durch** eine Nutzerschnittstelle (9-2), wobei die Nutzerschnittstelle (9-2) dazu eingerichtet ist, einen Startbefehl (24) als Eingabe zu erfassen, wobei die Steuereinrichtung (6) dazu eingerichtet ist, das Erfassen der Abbildungen (21l, 21r) und das Schätzen der optimalen Mikroskopparameter (40) und/oder der Änderung (41) der Mikroskopparameter (40) und/oder der Steuerbefehle (42) und das Ansteuern zu starten, wenn der Startbefehl (24) erfasst wurde.

## Claims

1. Method for operating a surgical microscope (1), wherein a left-side image representation (211) of a capture region (10) of the surgical microscope (1) is captured by means of a left-side camera (2) of the surgical microscope (1),
wherein a right-side image representation (21r) of the capture region (10) is captured by means of a right-side camera (3) of the surgical microscope (1),
wherein the captured left-side image representation (211) and the captured right-side image representation (21r) are fed as input data to at least one trained machine learning method (30) and/or computer vision evaluation method that is made available by means of a control device (6) of the surgical microscope (1), and
wherein, by means of the at least one trained machine learning method (30) and/or computer vision evaluation method and by using the captured image representations (211, 21r) as a starting point, a surgical target object (20) in the captured image representations (211, 21r) is identified, wherein a type of the target object and a position of the target object (20) are determined in the image representations (211, 21r) during the identification of the target object (20), and wherein optimum microscope parameters (40) and/or a change (41) in microscope parameters (40) and/or control commands (42) for an actuator system (5) of the surgical microscope (1) are estimated for the identified target object (20),
wherein control commands (42) for the actuator system (5) of the surgical microscope (1) are generated by means of the control device (6) from the estimated optimum microscope parameters (40) and/or the estimated change (41) in microscope parameters (41), and/or wherein the actuator system (5) is controlled according to the generated and/or estimated control commands (42), with the result that the microscope parameters for the surgical microscope are configured optimally with regards to the identified surgical target object, wherein the at least one trained machine learning method (30) and/or computer vision evaluation method estimates and provides a confidence value for each estimated output, wherein the output comprises the identified surgical target object (20), wherein the confidence value provided is compared with a specified threshold value (44), wherein the method is aborted if the confidence value provided is below the specified threshold value (44).

2. Method according to Claim 1, **characterized in that** microscope parameters (43) set during the capture of the image representations (211, 21r) are also additionally fed as input data to the at least one trained machine learning method (30).

3. Method according to either of the preceding claims, **characterized in that** a type (22) of a surgery and/or a phase (23) of the surgery are detected and/or received and fed as input data to the trained machine learning method (30).

4. Method according to any of the preceding claims, **characterized in that** a type (22) of a surgery and/or a phase (23) of the surgery are detected and/or received, wherein the at least one trained machine learning method (30) and/or computer vision evaluation method is selected from a plurality of trained machine learning methods (30) and/or computer vision evaluation methods depending on the type (22) of the surgery and/or the phase (23) of the surgery, wherein the selected at least one trained machine learning method (30) and/or computer vision evaluation method is used.

5. Method according to any of the preceding claims, **characterized in that** a start command (24) is detected as input, wherein the capture of the image representations (211, 21r), the identification and the estimation of the optimum microscope parameters (40) and/or the change (41) in microscope parameters (40) and/or control commands (42) and the controlling are started when the start command (24) has been detected.

6. Method according to any of the preceding claims, **characterized in that** the at least one trained machine learning method (30) comprises at least one trained neural network (31).

7. Method according to any of the preceding claims, **characterized in that** the identification of the surgical target object (20) takes place by means of the at least one trained machine learning method (30) and/or computer vision evaluation method, and the estimation of the optimum microscope parameters (40) and/or the change (41) in microscope parameters (40) and/or control commands (42) for the actuator system (5) takes place on the basis of a single captured left-side image representation (211) and a single captured right-side image representation (21r) .

8. Surgical microscope (1) comprising:
a left-side camera (2) adapted to capture a left-side image representation (211) of a capture region (10) of the surgical microscope (1),
a right-side camera (3) adapted to capture a right-side image representation (21r) of the capture region (10), an actuator system (5) adapted to set a configuration of the surgical microscope (1) according to microscope parameters (40), and
a control device (6), wherein the control device (6) is adapted to make available at least one trained machine learning method (30) and/or computer vision evaluation method, to feed the captured left-side image representation (211) and the captured right-side image representation (21r) as input data to the at least one trained machine learning method (30) and/or computer vision evaluation method,
wherein the at least one trained machine learning method (30) and/or computer vision evaluation method is adapted and/or trained, using the captured image representations (211, 211) as a starting point, to identify a surgical target object (20) in the captured image representations (211, 21r) and to determine a type of the target object (20) and a position of the target object (20) in the image representations (211, 21r) during the identification of the target object, and to estimate and provide herefor optimum microscope parameters (40) and/or a change (41) in microscope parameters (40) and/or control commands (42) for the actuator system (5),
wherein the control device (6) is further adapted to generate control commands (42) for the actuator system (5) of the surgical microscope (1) from the estimated optimum microscope parameters (40) and/or the estimated change (41) in microscope parameters (40) and/or to control the actuator system (5) of the surgical microscope (1) according to the generated and/or estimated control commands (42), with the result that the microscope parameters for the surgical microscope are configured optimally with regards to the identified surgical target object, wherein the at least one trained machine learning method (30) and/or computer vision evaluation method estimates and provides a confidence value for each estimated output, wherein the output comprises the identified surgical target object (20), wherein the control device (6) is further adapted to compare the confidence value provided with a specified threshold value (44) and to abort the method if the confidence value provided is below the specified threshold value (44).

9. Surgical microscope (1) according to Claim 8, **characterized by** at least one interface (9-1, 9-2), wherein the at least one interface (9-1, 9-2) is adapted to detect and/or receive a type (22) of a surgery and/or a phase (23) of the surgery, wherein the control device (6) is further adapted to feed the type (22) of the surgery and/or the phase (23) of the surgery as input data to the at least one trained machine learning method (30) and/or to select the at least one trained machine learning method (30) and/or computer vision evaluation method from a plurality of trained machine learning methods (30) and/or computer vision evaluation methods depending on the type (22) of the surgery and/or the phase (23) of the surgery and to make available the selected at least one trained machine learning method (30) and/or computer vision evaluation method for use.

10. Surgical microscope (1) according to Claim 8 or 9, **characterized by** a user interface (9-2), wherein the user interface (9-2) is adapted to capture a start command (24) as input, wherein the control device (6) is adapted to start the capture of the image representations (211, 21r) and the estimation of the optimum microscope parameters (40) and/or the change (41) in microscope parameters (40) and/or control commands (42) and the controlling when the start command (24) has been detected.

## Revendications

1. Procédé de fonctionnement d'un microscope chirurgical (1),
une image côté gauche (211) d'une zone de capture (10) du microscope chirurgical (1) étant capturée au moyen d'une caméra côté gauche (2) du microscope chirurgical (1),
une image côté droit (21r) de la zone de capture (10) étant capturée au moyen d'une caméra côté droit (3) du microscope chirurgical (1),
l'image côté gauche capturée (211) et l'image côté droit capturée (21r) étant amenées en tant que données d'entrée à au moins un procédé d'apprentissage automatique entraîné (30) et/ou un procédé d'évaluation de vision par ordinateur mis en œuvre par un dispositif de commande (6) du microscope chirurgical (1), et
un objet cible chirurgical (20) étant identifié dans les images capturées (211, 21r) à l'aide dudit au moins un procédé d'apprentissage automatique entraîné (30) et/ou procédé d'évaluation de vision par ordinateur sur la base des images capturées (211, 21r), un type d'objet cible et une position de l'objet cible (20) étant déterminés dans les images (211, 21r) lors de l'identification de l'objet cible (20), et des paramètres de microscope optimaux (40) et/ou une modification (41) de paramètres de microscope (40) et/ou des instructions de commande (42) destinées à un système d'actionneurs (5) du microscope chirurgical (1) étant estimés pour l'objet cible identifié (20),
des instructions de commande (42) destinées au système d'actionneurs (5) du microscope chirurgical (1) étant générées au moyen du dispositif de commande (6) à partir des paramètres de microscope optimaux estimés (40) et/ou de la modification estimée (41) des paramètres de microscope (41) et/ou le système d'actionneurs (5) étant commandé conformément aux instructions de commande générées et/ou estimées (42), de sorte que les paramètres du microscope chirurgical soient configurés de manière optimale pour l'objet cible chirurgical identifié,
l'au moins un procédé d'apprentissage automatique entraîné (30) et/ou le procédé d'évaluation de vision par ordinateur estimant et fournissant une valeur de confiance pour chaque sortie estimée, la sortie comprenant l'objet cible chirurgical identifié (20), la valeur de confiance fournie étant comparée à une valeur seuil spécifiée (44), le procédé étant terminé lorsque la valeur de confiance fournie est inférieure à la valeur seuil spécifiée (44).

2. Procédé selon la revendication 1, **caractérisé en ce que** des paramètres de microscope (43) réglés lors de la capture des images (211, 21r) étant en outre amenés également comme données d'entrée à l'au moins un procédé d'apprentissage automatique entraîné (30).

3. Procédé selon l'une des revendications précédentes, **caractérisé en ce qu'**un type (22) d'une intervention chirurgicale et/ou une phase (23) de l'intervention chirurgicale sont détectés et/ou reçus et amenés comme données d'entrée au procédé d'apprentissage automatique entraîné (30).

4. Procédé selon l'une des revendications précédentes, **caractérisé en ce qu'**un type (22) d'intervention chirurgicale et/ou une phase (23) de l'intervention chirurgicale sont acquis et/ou reçus, l'au moins un procédé d'apprentissage automatique entraîné (30) et/ou procédé d'évaluation de vision par ordinateur étant sélectionnés en fonction du type (22) d'intervention chirurgicale et/ou de la phase (23) de l'intervention chirurgicale parmi plusieurs procédés d'apprentissage automatique entraînés (30) et/ou procédés d'évaluation de vision par ordinateur, l'au moins un procédé d'apprentissage automatique entraîné (30) et/ou procédé de vision par ordinateur sélectionné étant utilisé.

5. Procédé selon l'une des revendications précédentes, **caractérisé en ce qu'**une instruction de démarrage (24) est acquise en entrée, la capture des images (211, 21r), l'identification et l'estimation des paramètres de microscope optimaux (40) et/ou la modification (41) des paramètres de microscope (40) et/ou des instructions de commande (42) et la commande étant démarrées lorsque l'instruction de démarrage (24) a été acquise.

6. Procédé selon l'une des revendications précédentes, **caractérisé en ce que** l'au moins un procédé d'apprentissage automatique entraîné (30) comprend au moins un réseau neuronal entraîné (31).

7. Procédé selon l'une des revendications précédentes, **caractérisé en ce que** l'identification de l'objet cible chirurgical (20) au moyen de l'au moins un procédé d'apprentissage automatique entraîné (30) et/ou procédé d'évaluation de vision par ordinateur et l'estimation des paramètres de microscope optimaux (40) et/ou de la modification (41) des paramètres de microscope (40) et/ou des instructions de commande (42) destinées au système d'actionneurs (5) sont effectuées sur la base d'une seule image côté gauche capturée (211) et d'une seule image côté droit capturée (21r).

8. Microscope chirurgical (1), comprenant :
une caméra côté gauche (2), conçue pour capturer une image côté gauche (211) d'une zone de capture (10) du microscope chirurgical (1),
une caméra côté droit (3), conçue pour capturer une image côté droit (21r) de la zone de capture (10),
un système d'actionneurs (5), conçu pour définir une configuration du microscope chirurgical (1) en fonction de paramètres de microscope (40), et
un dispositif de commande (6), le dispositif de commande (6) étant conçu pour fournir au moins un procédé d'apprentissage automatique entraîné (30) et/ou procédé d'évaluation de vision par ordinateur, amener l'image côté gauche capturée (211) et l'image côté droit capturée (21r) comme données d'entrée à l'au moins un procédé d'apprentissage automatique entraîné (30) et/ou procédé d'évaluation de vision par ordinateur,
l'au moins un procédé d'apprentissage automatique entraîné (30) et/ou procédé d'évaluation de vision par ordinateur étant conçus et/ou entraînés pour identifier un objet cible chirurgical (20) dans les images capturées (211, 211) sur la base des images capturées (211, 21r), et déterminer lors de l'identification de l'objet cible un type d'objet cible (20) et une position de l'objet cible (20) dans les images (211, 21r), et pour estimer et fournir, pour l'objet cible identifié (20), des paramètres de microscope optimaux (40) et/ou une modification (41) des paramètres de microscope (40) et/ou des instructions de commande (42) destinées au système d'actionneurs (5),
le dispositif de commande (6) étant en outre conçu pour générer des instructions de commande (42) destinées au système d'actionneurs (5) du microscope chirurgical (1) à partir des paramètres de microscope optimaux estimés (40) et/ou de la modification estimée (41) des paramètres de microscope (40) et/ou pour commander le système d'actionneurs (5) du microscope chirurgical (1) conformément aux instructions de commande générées et/ou estimées (42), de sorte que les paramètres du microscope chirurgical soient configurés de manière optimisée pour l'objet cible chirurgical identifié,
l'au moins un procédé d'apprentissage automatique entraîné (30) et/ou procédé d'évaluation de vision par ordinateur estimant et fournissant une valeur de confiance pour chaque sortie estimée, la sortie comprenant la cible chirurgicale identifiée (20),
le dispositif de commande (6) étant en outre conçu pour comparer la valeur de confiance fournie à une valeur seuil spécifiée (44) et pour interrompre le procédé si la valeur de confiance fournie est inférieure à la valeur seuil spécifiée (44).

9. Microscope chirurgical (1) selon la revendication 8, **caractérisé par** au moins une interface (9-1, 9-2), l'au moins une interface (9-1, 9-2) étant conçue pour acquérir et/ou recevoir un type (22) d'intervention chirurgicale et/ou une phase (23) de l'intervention chirurgicale,
le dispositif de commande (6) étant en outre conçu pour amener le type (22) d'intervention chirurgicale et/ou la phase (23) de l'intervention chirurgicale comme données d'entrée à l'au moins un procédé d'apprentissage automatique entraîné (30) et/ou pour sélectionner l'au moins un procédé d'apprentissage automatique entraîné (30) et/ou procédé d'évaluation de vision par ordinateur parmi plusieurs procédés d'apprentissage automatique entraînés (30) et/ou procédés d'évaluation de vision par ordinateur en fonction du type (22) d'intervention chirurgicale et/ou de la phase (23) de l'intervention chirurgicale et pour fournir l'au moins un procédé d'apprentissage automatique sélectionné (30) et/ou procédé d'évaluation de vision par ordinateur à des fins d'utilisation.

10. Microscope chirurgical (1) selon la revendication 8 ou 9, **caractérisé par** une interface utilisateur (9-2), l'interface utilisateur (9-2) étant conçue pour acquérir une instruction de démarrage (24) comme entrée, le dispositif de commande (6) étant pour démarrer la capture des images (211, 21r) et l'estimation des paramètres de microscope optimaux (40) et/ou la modification (41) des paramètres de microscope (40) et/ou des instructions de commande (42) et la commande lorsque l'instruction de démarrage (24) a été acquise.
